# EUROPEAN PATENT SPECIFICATION

(11) **EP 2 550 357 B1**
(45) Date of publication and mention of the grant of the patent: **14.09.2016**
(21) Application number: 11721134.2
(22) Date of filing: 11.05.2011
(51) Int. Cl.: C12M 1/00, C12M 3/06, C12M 1/34, C12M 1/12

(54) **CELL-CULTURE-BAG**
ZELLKULTURBEUTEL
SAC DE CULTURE DE CELLULES

(30) Priority: 12.05.2010 EP 10162688; 16.08.2010 EP 10172939
(43) Date of publication of application: 30.01.2013
(73) Proprietor: Xpand Biotechnology BV, 3723 MB Bilthoven (NL)
(72) Inventor: DIJKHUIZEN BORGART, Elise Leonore Isolde, NL-3431 SG Nieuwegein (NL); BRACKE, Madelon Sophia George Maria, NL-3708 GA Zeist (NL); DE BRUIJN, Joost Dick, NL-3817 JL Amersfoort (NL)
(74) Representative: V.O.
(86) International application number: PCT/NL2011/050322
(87) International publication number: WO 2011/142670

(56) References cited:
- WO-A1-2008/060037
- WO-A1-2009/139703
- US-A1- 2005 244 963
- HUNG SHIH-CHIEH ET AL: "Isolation and characterization of size-sieved stem cells from human bone marrow", STEM CELLS (MIAMISBURG), vol. 20, no. 3, 2002, pages 249-258, XP009054150, ISSN: 1066-5099
- SCHOP D ET AL: "Expansion of human mesenchymal stromal cells on microcarriers: growth and metabolism", JOURNAL OF TISSUE ENGINEERING AND REGENERATIVE MEDICINE, JOHN WILEY & SONS, US LNKD- DOI:10.1002/TERM.224, vol. 4, no. 2, 1 March 2010 (2010-03-01), pages 131-140, XP009135743, ISSN: 1932-6254 [retrieved on 2009-10-19]
- BING R J ET AL: "The use of microcarrier beads in the production of endothelium-derived relaxing factor by freshly harvested endothelial cells", TISSUE AND CELL, CHURCHILL LIVINGSTONE MEDICAL JOURNALS, EDINBURGH, GB LNKD- DOI:10.1016/0040-8166(91)90070-A, vol. 23, no. 2, 1 January 1991 (1991-01-01), pages 151-159, XP023044120, ISSN: 0040-8166 [retrieved on 1991-01-01]
- OH S K W ET AL: "Long-term microcarrier suspension cultures of human embryonic stem cells", STEM CELL RESEARCH,, vol. 2, no. 3, 1 May 2009 (2009-05-01), pages 219-230, XP026087759, ISSN: 1873-5061 [retrieved on 2009-03-04]

## Description

The present invention relates to a system for expanding stem cells.

In order to treat a whole range of tissue disorders from cardiovascular disease to bone defects, it is known to use fresh bone marrow as a source of adult stem cells (mesenchymal stromal cells/MSCs), culture expand those cells into clinically required quantities (200-800 million cells) and then use them for autologous implantation as part of tissue replacement treatment.

WO2009/139703 discloses a method of expanding cells such as MSCs in a plastic bag reactor. The method uses a purified patient tissue sample of cells which are pre-cultured in a T-flask to achieve enough MSC quantity to be transferred to the plastic bag reactor. Within the plastic bag reactor, the cells are adhered to microcarriers, and culture expanded. The pre-culturing step brings with it several drawbacks. The need for the T-flask introduces material costs. The need to perform the steps of purifying and culturing the cells in the T-flask, and then perform the steps of collecting and transferring the pre-seeded cells to the plastic bag reactor introduces manpower costs for highly-trained and thus expensive technicians. Further, the transferring step inherently carries with it the risk of contamination and makes the method poorly suited for use in the field (i.e. outside of an R&D setting).

With this background in mind, according to a first aspect, the present invention provides a system for expanding stem cells as defined in claim 1.

The cell-culture-bag according to the present invention is structured such that it is able to internally host all the operations necessary for the expansion of stem cells from a crude biopsy. The cell-culture-bag provides a closed bioreactor environment that makes it suitable for use in the field, for example, in a hospital.

In order to allow passage of red blood cells and block passage of stem cells from the first outlet, the filter arrangement filters the fluid path from the chamber to the first outlet with a relatively fine mesh size of preferably 8-20µm, more preferably 8-12µm and most preferably 8-10µm.

In order to block passage of microcarriers from the second outlet, the filter arrangement filters the fluid path from the chamber to the third outlet with a relatively coarse mesh size of preferably 50-100µm, more preferably 50-70µm, and most preferably 60µm.

The cell-culture-bag may comprise a third outlet, the filter arrangement being constructed to block the passage of at least microcarriers and optionally stem cells from the third outlet.

In order to block passage of microcarriers from the third outlet, the filter arrangement filters the fluid path from the chamber to the third outlet with a relatively coarse mesh size of preferably 50-100µm, more preferably 50-70µm, and most preferably 60µm. When optionally the passage of stem cells from the third outlet is also blocked, the filter arrangement filters the fluid path from the chamber to the third outlet with a relatively fine mesh size of preferably 8-20µm, more preferably 8-12µm and most preferably 8-10µm.

In a preferred embodiment, the filter arrangement comprises a filter member that is associated with a said outlet and joins to the portion of the outer wall that surrounds the said outlet so as to sealingly enclose said outlet. The filter member is preferably joined to the inside surface of the outer wall. Alternatively, it may be joined to the outside surface of the outer wall. In such a case, a further cover member is also attached to the outside surface of the outer wall sealingly encloses the filter member. The filter member may be in the form of a fabric.

Preferably, the area of the filter member is substantially greater than the (cross-sectional) area of the associated outlet. A small outlet is advantageous in terms of fluid transport, and making the area of the filter member large assists in avoiding clogging.

Preferably, the join between the filter member and the outer wall includes a protuberance that serves to create a small clearance space between the filter member and the outer wall. In one embodiment, the protuberance is formed in the outer wall by a heat sealing operation that creates the join. The small clearance space assists in preventing the filter member from becoming pressed flat against the outer wall. In this way, the filter member maintains a large active area during filtering.

Preferably, each of said outlets has a respective filter member. In other embodiments, one filter member may be associated with more than one outlet and be arranged so as to sealingly enclose said more than one outlet.

Preferably, each of said outlets and the inlet has an associated coupling for connection to a conduit of the perfusion apparatus.

Preferably, the filter arrangement/filter members are made from a material that is less attractive for attachment to the stem cells than the microcarriers and/or non-toxic to the stem cells.

In the context of the present invention, when separate filters are both referred to as "coarse" or "fine" within the same bag, they need not be the same mesh size.

A system according to the present invention carries out the expansion of stem cells from a crude biopsy within a single bioreactor environment provided by the cell-culture-bag and thereby avoids the above-mentioned drawbacks of the method of WO2009/139703.

According to a third aspect, the present invention may provide a system for expanding cells from a crude biopsy, comprising:
a cell-culture-bag comprising a chamber; a first inlet, and a seeding/expansion outlet, and a collection outlet, each in fluid communication with the chamber;
a perfusion apparatus connected to the first inlet, and the seeding/expansion and collection outlets;
wherein the perfusion apparatus has a seeding mode in which the first inlet and the seeding/expansion outlet are in operative connection with a seeding circuit of the perfusion apparatus, and a seeding operation is performed on stem cells within the chamber;
wherein the perfusion apparatus has an expansion mode in which the first inlet and the seeding/expansion outlet are in operative connection with an expansion circuit of the perfusion apparatus, and an expansion operation is performed on stem cells within the chamber;
wherein the perfusion apparatus has a harvesting mode in which the first inlet is in operative connection with a harvesting section of the perfusion apparatus, and an harvesting operation is performed on stem cells within the chamber;
wherein the perfusion apparatus has a collection mode in which the collection outlet is in operative connection with a collection section of the perfusion apparatus, and harvested stem cells exit from the collection outlet.

According to a fourth aspect, the present invention may provide a system for expanding cells from a crude biopsy, comprising:
a cell-culture-bag comprising a chamber; a first inlet, and a seeding outlet, an expansion outlet, and a collection outlet, each in fluid communication with the chamber;
a perfusion apparatus connected to the first inlet, and the seeding, expansion and collection outlets;
wherein the perfusion apparatus has a seeding mode in which the first inlet and the seeding outlet are in operative connection with a seeding circuit of the perfusion apparatus, and a seeding operation is performed on stems cells within the chamber;
wherein the perfusion apparatus has an expansion mode in which the first inlet and the expansion outlet are in operative connection with an expansion circuit of the perfusion apparatus, and an expansion operation is performed on stem cells within the chamber;
wherein the perfusion apparatus has a harvesting mode in which the first inlet is in operative connection with a harvesting section of the perfusion apparatus and a harvesting operation is performed on stem cells within the chamber; and
wherein the perfusion apparatus has a collection mode in which the collection outlet is in operative connection with a collection section of the perfusion apparatus, and harvested stem cells exit from the collection outlet.

System according to the third or fourth aspects of the present invention carry out the expansion of stem cells from a crude biopsy within a single bioreactor environment provided by the cell-culture-bag and thereby avoids the above-mentioned drawbacks of the method of WO2009/139703.

A biopsy is a sample of cells or tissue removed from a living being. A biopsy may be an entire lump or area that is removed. When a sample of tissue of fluid is removed with a needed in such a way that cells are removed without preserving the histological architecture of the tissue cells, the procedure is called a needle aspiration biopsy, or aspirate. In the context of the present invention, the terms biopsy and aspirate are used interchangeably. A biopsy or aspirate may be of any size, depending on the amount of cells needed and the amount of cells a living being may be able to donate. Biopsies and aspirates may be further purified or modified, e.g. a filter step or gradient purification to remove certain ingredients of the biopsy or aspirate, e.g. certain cells or proteins. In some cases additional tissue other than the desired biopsy tissue is present in the biopsy or aspirate due to the technique of extracting the biopsy.

For example when obtaining a bone marrow biopsy one has to go through the skin and possible a fat layer and then through the bone tissue in order to reach the bone marrow. The bone marrow biopsy then may contain bone chips and lumps of fatty tissue or cell aggregates. The additional tissue is often removed from the biopsy; however the cellular composition of the biopsy is still the same as the tissue in the living being from which it is withdrawn. Biopsies wherein only the additional tissue is removed and the cellular composition of the biopsy is the same as the cellular composition of the living tissue are referred to as cellular or crude biopsies. For example in the case of bone marrow aspirate, the bone chips, fatty tissue or cell aggregates are removed by a course filter to make sure that the cellular composition of the aspirate is the same as the bone marrow in the living being it was extracted from. This is called a cellular or crude bone marrow aspirate. A cellular or crude bone marrow aspirate contains a mixture of cells (e.g. red blood cells, platelets , white blood cells, fat cells and Mesenchymal stem cell) which is comparable to the bone marrow as it is present in the organism. In the context of the present invention, crude or cellular biopsy means a biopsy that has the same composition of cells as it has in the organism. For example, a crude bone marrow biopsy, where only the large bone chips and fat tissue is removed but has otherwise the same cellular make up as bone marrow in the organism may be placed into the cell-culture-bag of the present invention as the starting material. No other treatment is necessary. No pre-washing or pre-culturing is needed. The present invention is preferably concerned with the expansion of adult human stem cells.

Exemplary embodiments of the present invention are hereinafter described with reference to the accompanying drawings, in which:
Figure 1 shows a system, including a first cell-culture-bag, expanding stem cells from bone marrow aspirate, which does not form part of the present invention;
Figure 2 shows the operative parts of the Figure 1 system when it is operating in a seeding mode;
Figure 3 shows the operative parts of the Figure 1 system when it is operating in an expansion mode;
Figure 4 shows the operative parts of the Figure 1 system when it is operating in a harvesting mode and/or a collection mode;
Figure 5 shows a view from above of a second cell-culture-bag, which does not form part of the present invention;
Figure 6 shows a cross-sectional view along the line X-X in Figure 5;
Figure 7 shows a view from above a fourth cell-culture-bag; and
Figures 8(a), (b), (c) show side views of the fourth cell-culture-bag in use.

Throughout the following description the same or corresponding parts have been given the same or corresponding reference numerals.

Stem cells: the classical definition of a stem cell requires that it possess two properties: Self-renewal - the ability to go through numerous cycles of cell division while maintaining the undifferentiated state. Potency - the capacity to differentiate into specialized cell types. In the strictest sense, this requires stem cells to be either totipotent, pluripotent or multipotent- to be able to give rise to any mature cell type, although unipotent stem cells have also been described.

The two broad types of mammalian stem cells are: embryonic stem cells that are isolated from the inner cell mass of blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin, or intestinal tissues.

Totipotent (a.k.a omnipotent) stem cells can differentiate into embryonic and extraembryonic cell types. Such cells can construct a complete, viable, organism. These cells are produced from the fusion of an egg and sperm cell. Cells produced by the first few divisions of the fertilized egg are also totipotent. Pluripotent stem cells are the descendants of totipotent cells and can differentiate into nearly all cells i.e. cells derived from any of the three germ layers.

Multipotent stem cells can differentiate into a number of cells, but only those of a closely related family of cells, usually within the germ layer that the mesenchymal stem cell originates from.

Unipotent cells can produce only one cell type, their own, but have the property of self-renewal which distinguishes them from non-stem cells (e.g. skin stem cells).

Adult stem cells refer to any cell which is found in a developed organism that has two properties: the ability to divide and create another cell like itself and also divide and create a cell more differentiated than itself. Adult stem cells can be found in children, as well as adults. Pluripotent adult stem cells are rare and generally small in number but can be found in a number of tissues including umbilical cord blood and bone marrow. Most adult stem cells are lineage-restricted (multipotent) and are generally referred to by their germ-layer or tissue origin (mesenchymal stem cell, adipose-derived stem cell, endothelial stem cell, etc.). Adult stem cell treatments have been successfully used for many years to treat leukemia and related bone/blood cancers through bone marrow transplants. Adult stem cells are also used in veterinary medicine to treat tendon and ligament injuries in horses. Adult stem cells can be obtained from the intended recipient, (an autograft) the risk of rejection is essentially non-existent in these situations.

Hematopoietic stem cells (HSCs) are non-adherent multipotent stem cells that give rise to all the blood cell types including myeloid (monocytes and macrophages, neutrophils, basophils, eosinophils, erythrocytes, megakaryocytes/platelets, dendritic cells), and lymphoid lineages (T-cells, B-cells, NK-cells). Hematopoietic stem cells are found in the bone marrow of adults. Cells can be obtained directly by removal from the hip using a needle and syringe, or from the blood following pre-treatment with cytokines, such as G-CSF (granulocyte colony-stimulating factors), that induce cells to be released from the bone marrow compartment. Other sources for clinical and scientific use include umbilical cord blood, placenta, mobilized peripheral blood. For experimental purposes, fetal liver, fetal spleen, and AGM (Aorta-gonad-mesonephros) of animals are also useful sources of HSCs.

Mesenchymal stem cells (MSCs) are of stromal origin and may differentiate into a variety of tissues. MSCs have been isolated from placenta, adipose tissue, lung, bone marrow and blood, Wharton's jelly from the umbilical cord, and teeth (perivascular niche of dental pulp and periodontal ligament). Cell types that MSCs have been shown to differentiate into in vitro or in vivo include osteoblasts, chondrocytes and adipocytes. MSC are sometimes referred to Marrow Stromal Cell or Mesenchymal Stromal Cell and have been used interchangeably.

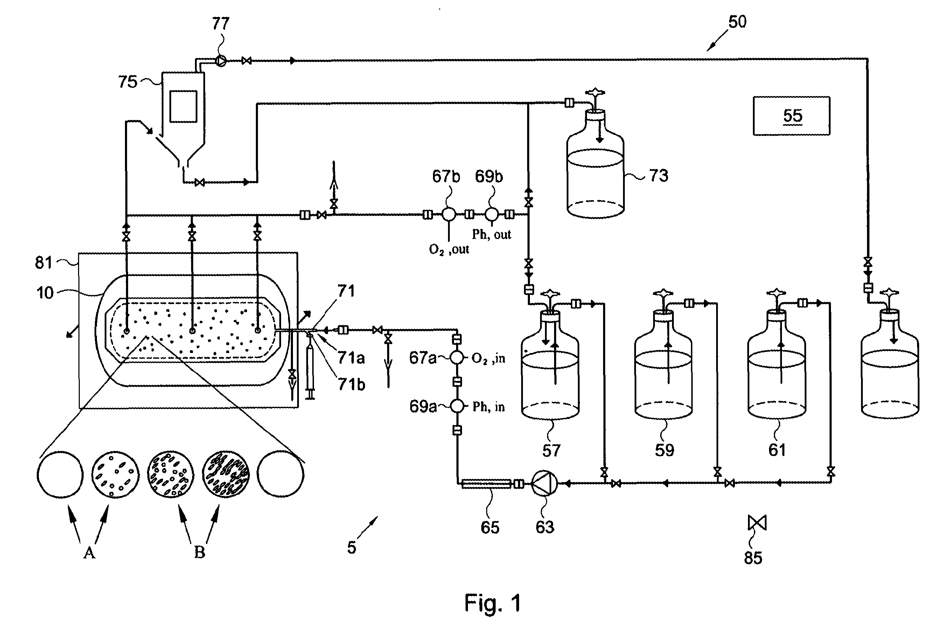

Figure 1 shows a system 5 for expanding adult human stem cells from bone marrow aspirate into the quantities required clinically. The system 5 comprises a first cell-culture-bag 10 that is connected up to a perfusion apparatus 50.

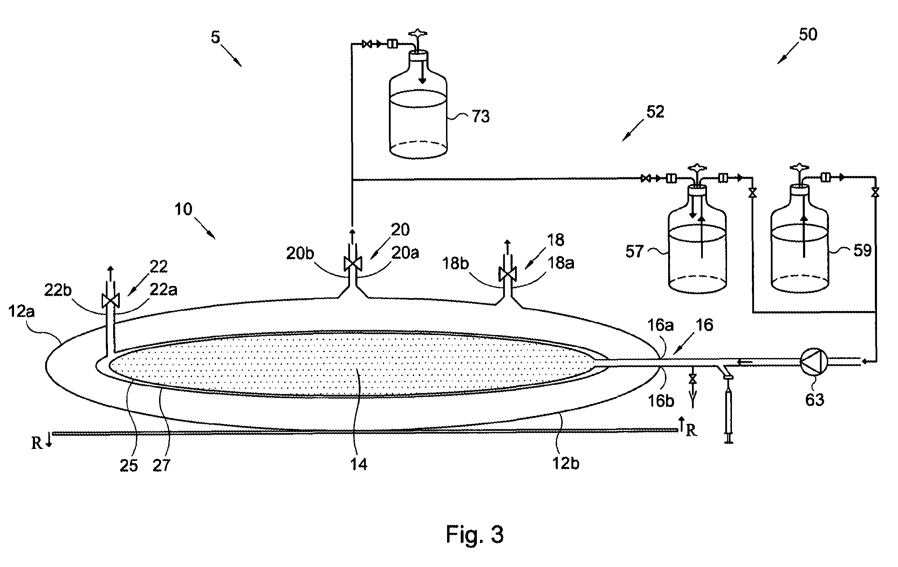

Referring to Figure 3, the first cell-culture-bag 10 is disposable and comprises an outer wall 12, comprising an upper wall portion 12a and a lower wall portion 12b. The cell-culture-bag 10 comprises a first inlet port 16, comprising a first inlet 16a and a first inlet coupling 16b, a first/seeding outlet port 18, comprising a first/seeding outlet 18a and a first/seeding coupling 18b, a third/expansion outlet port 20, comprising a third/expansion outlet 20a and a third/expansion coupling 20b, and a second/collection outlet port 22, comprising a second/collection outlet 22a and a second/collection outlet coupling 22b. The cell-culture-bag 10 further comprises an inner filter bag 25 having a relatively coarse mesh size of 100µm that is intended to block the passage of microcarriers, and a surrounding outer filter bag 27 having a relatively fine mesh size of 8µm that is intended to allow the passage of red blood cells but block the passage of MSCs. The filter bags 25, 27 are arranged such that the inlet port 16 is in fluid communication with an internal chamber 14 within the interior of the inner filter bag 25.

The seeding and expansion ports 18, 20 are separated from the chamber 14 by both filter bags 25, 27. The collection coupling 22b passes through an aperture in the outer filter bag 27, whereby the collection port 22 is separated from the chamber 14 by only the inner filter bag 25.

Referring to Figure 1, the perfusion apparatus 50 comprises a controller 55 for co-ordinating the overall operation of the system.

The perfusion apparatus 50 further comprises an expansion medium vessel 57 containing fresh nutrient, a microcarrier suspension vessel 59 containing Cytodex microcarriers (150µm), and a cell dissociating solution vessel 61 containing cell dissociating solution, which are each coupled in parallel to the inlet port 16. A pump 63 for pumping the media from the vessels 57, 59, 61 to the inlet port 16, a control device 65, for example, an oxygenator for saturating the medium with the required concentrations of oxygen and carbon dioxide, an oxygen control flow cell 67a, and a ph control flow cell 69b are coupled in series downstream of the vessels 57, 59, 61. A conduit or tubing 71 provides fluid communication between the flow cell 69 and the inlet port 16 where it connects with the inlet coupling 16b. The conduit 71 comprises a side conduit 71a partway therealong. The side conduit 71a comprises a filter 71b having a relatively coarse mesh size of 100µm. The side conduit 71a is adapted to permit crude aspirate to be injected into the conduit 71 and thence to enter into the cell-culture-bag 10 via the inlet port 16. In other embodiments (not shown), the crude aspirate may be injected into the cell-culture-bag via a second inlet port. A relatively coarse mesh filter may be integrated into the bag or be located external to the bag, for example, in the conduit that attaches to the second inlet port.

The perfusion apparatus 50 further comprises an oxygen control flow cell 67b, and a ph control flow cell 69b which are coupled between the outlet ports 18, 20, 22 and the expansion medium vessel 57 and a waste medium vessel 73 arranged in parallel. Also coupled to the outlet ports 18, 20, 22 is a bio-separation device 75 operable by ultrasonic separation to concentrate an MSC suspension before pumping it by a pump 77 to a sterile clinical device 79 for subsequent treatment and use. The separation device 75 is also coupled to the waste medium vessel 73.

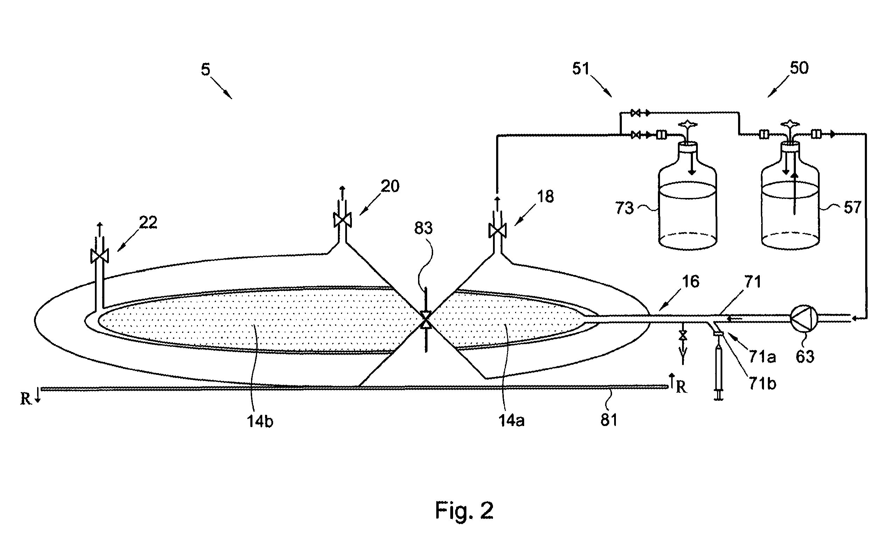

The perfusion apparatus further comprises a rocker device 81 to which the cell-culture-bag 10 is mounted. The rocker device 81 is capable of imparting an x-y 180° motion, as indicated by the arrows R, to the cell-culture-bag 10. The perfusion apparatus 50 further comprises a bag clamp device 83 (shown abstractly in Figure 2) operable to partition the chamber 14 into a smaller sub-chamber 14a that includes the first inlet 16a and the seeding outlet 18a, and a larger sub-chamber 14b that includes the expansion and collection outlets 20a, 22a. The perfusion apparatus 50 further comprises a plurality of circuit clamps 85 that can be moved between an open and closed condition so as to switch-in or switch-out the above-described parts of the system.

The above-described parts of the system are preferably controlled by the controller 55.

The operation of the system 5 in processing of MSCs from crude bone marrow aspirate and expanding them is now described with reference to Figures 2 to 4.

In an initial set-up mode, the perfusion apparatus 50 is operable to activate the bag clamp 83 from an inoperative position to the operative position in Figure 2 in which the chamber 14 is partitioned into the sub-chambers 14a, 14b. The perfusion apparatus 50 is further operable to pump microcarriers from the microcarrier suspension vessel 59 into the sub-chamber 14a. During the set-up mode, either before or after the introduction of microcarriers into the sub-chamber 14a, the user injects by syringe crude bone marrow aspirate into the sub-chamber 14a via the side conduit 71a. The coarse mesh filter 71b serves to block the passage of large blood clots and other large tissue particulates such as bone chips and the like.

Next, the perfusion apparatus 50 is switched into a seeding mode, as shown in Figure 2, in which the first inlet 16 and the seeding outlet port 18 are in operative connection with a seeding circuit 51 of the perfusion apparatus 50 that includes the expansion medium vessel 57 and the waste medium vessel 73. In this mode, medium is pumped through the sub-chamber 14a and back either into the expansion medium vessel 57 or into the waste medium vessel 73 and the rocker device 81 subjects the cell-culture-bag 10 to a gentle rocking motion to keep the microcarriers in suspension. Under these conditions, the MSCs of the bone marrow aspirate adhere to the microcarriers as illustrated by the arrows A in Figure 1. The outer filter bag 27 having a mesh size of 8µm, allows the red blood cells and other small cells to pass out of the seeding port 18, but blocks the passage of MSCs that have adhered to the microcarriers. It may be appreciated that the microcarriers are mainly blocked by the inner filter bag 25, and only those microcarriers that have broken into fragments are blocked by the outer filter bag 27.

Next, the perfusion apparatus 50 is switched into an expansion mode as shown in Figure 3. In this mode, the bag clamp 83 is released into an inoperative position, thereby restoring the chamber 14. In addition, the circuit clamps 85 are configured such that an expansion circuit 52 of the perfusion apparatus 50 is formed between the first inlet port 16 and the expansion port 20, that includes the expansion medium vessel 57, the microcarrier suspension vessel 59 and the waste medium vessel 73. The seeding and collection outlet ports 18, 22 are closed off. In this mode, a more vigorous rocking motion (than in the seeding mode) is required to prevent formation of microcarrier/cell aggregates. Under these conditions, the fresh nutrient being pumped from the expansion medium vessel 57 enables the cells to grow as illustrated by the arrows B in Figure 1. The adherent MSCs are retained in the chamber 14 by the filter bags 25,27.

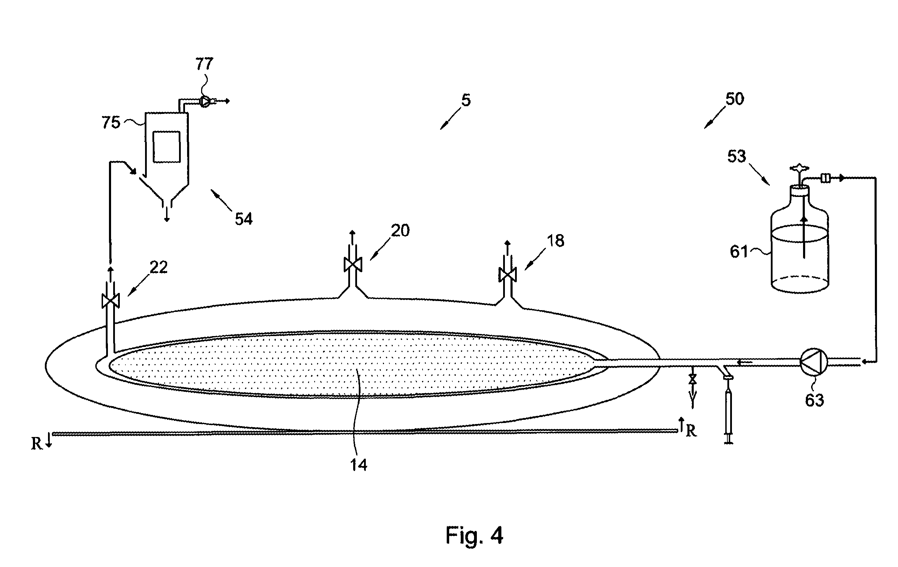

Referring to Figure 4, the perfusion apparatus 50 enters into a harvesting mode in which the first inlet port 16 is in operative connection with a harvesting section 53 of the perfusion apparatus 50 that includes the cell dissociating solution vessel 61. The seeding and expansion ports 18, 20 are closed off. In this mode, the cell disassociating solution is pumped into the chamber 14 and the rocker device 81 subjects the cell-culture-bag 10 to a relatively vigorous rocking motion in order to facilitate detachment of the MSCs from the microcarriers. Under these conditions, the MSCs become detached from the microcarriers.

Referring still to Figure 4, the perfusion apparatus 50 then enters into a collection mode in which the collection port 22 is in operative connection with a collection section 54 of the perfusion apparatus 50 that includes the bio-separator device 75. In this mode, the detached MSCs exit from the collection port 22 through the bag filter 25 and enter the bio-separator device 75 and the microcarriers are retained in the chamber 14 by the filter bag 25. In the bio-separator device 75 the MSC suspension is concentrated and then pumped into the sterile clinical device 79 (see Figure 1) for subsequent treatment and use.

Thus, the cell-culture-bag 10 is able to internally host each of the seeding, harvesting, and collection operations performed in the expansion of MSCs from crude biopsy.

The perfusion apparatus 50 can be in both the harvesting mode and collection mode simultaneously, and preferably, enters the collection mode partway through the harvesting operation.

The perfusion apparatus 50 may control the transition between one mode and the next according to parameters determined a priori. Alternatively, the perfusion apparatus 50 may take measurements to guide the transitions. In one embodiment, the perfusion apparatus comprises an optical sensor that enables the colour of the fluid substances within the sub-chamber 14a during the seeding operation to be measured. As the quantity of red blood cells decreases, so does the redness of the fluid substances. In this embodiment, the material from which the upper wall portion 12a is made must be sufficiently transparent or translucent.

In this patent specification, the system 5, apparatus 50 and bag 10 are performing a method that is more extensively disclosed in the applicant's copending application EP 10162688.5 filed on the 12 May 2010 and entitled "Expansion of cells in a disposable bioreactor", which is incorporated herein by reference. The system 5 and the apparatus 50 are capable of operating according to the method steps disclosed therein.

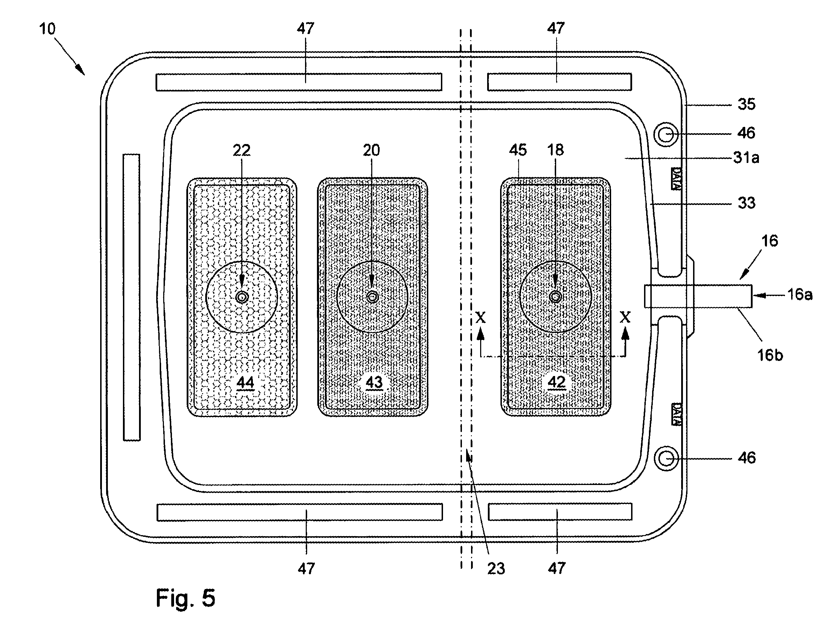

A second cell-culture-bag 10, intended for use in the Figure 1 system as a preferred alternative to the first cell-culture-bag, is shown in Figure 5.

The second cell-culture-bag 10 is disposable and comprises first and second, generally rectangular, sheets 31a, 31b of ethylene vinyl acetate (EVA) heat sealed along a first contour weld 33 inwardly spaced from the edges of the sheets 31a, 31b to define a chamber 14. A second contour weld 35 formed by heat sealing along the edges of the sheets 31a, 31b creates a looped skirt 37 between the first and second contour welds 33, 35. The second cell-culture-bag 10 further comprises a first inlet port 16 positioned on a central longitudinal axis of the bag. The first inlet port 16 comprises a first inlet coupling 16b that defines an inlet 16a providing communication between the exterior of the bag to the chamber 14. The first inlet coupling 16b is held fixedly in place between the sheets 31a, 31b by the contour welds 33, 35. The second cell-culture-bag 10 further comprises a first/seeding outlet port 18, a third/expansion outlet port 20, and a second/collection outlet port 22 positioned at the first sheet 31a on the central longitudinal axis of the bag at spaced intervals from the first inlet port 16. The ports have been positioned such that the spacing between the seeding port 18 and the expansion port 20 is much greater than that between the expansion port 20 and the collection port 22 in order to create a distinct filter-free region 23 suitable for being clamped.

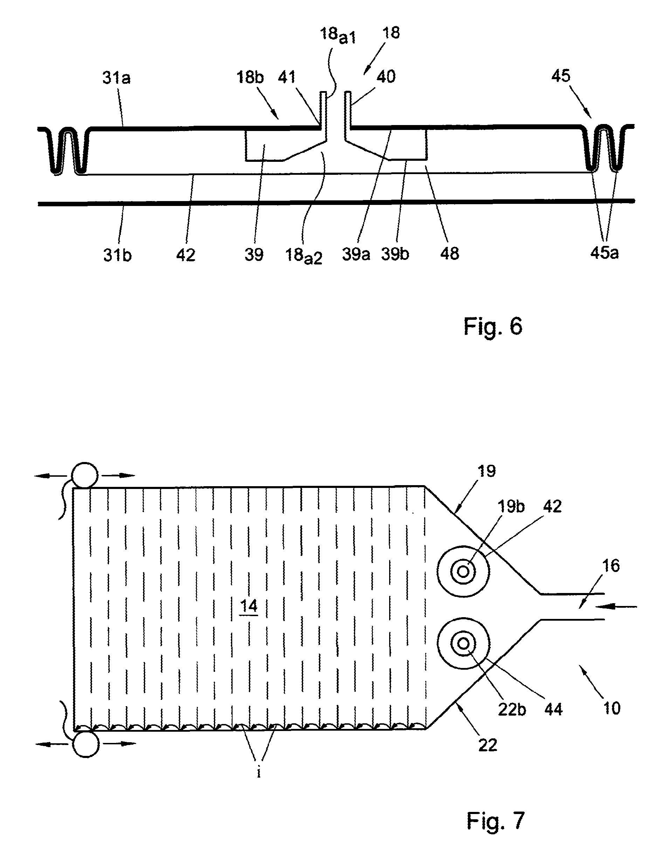

Each port 18, 20, 22 comprises a coupling 18b, 20b, 22b that defines an outlet 18a, 18b, 18c. Each port is the same in structure, and, for the purpose of explanation, the seeding port 18 is shown in more detail in Figure 6. Referring to Figure 6, the seeding port coupling 18b comprises a squat, generally cylindrical base portion 39 having an upper surface 39a and a lower surface 39b, and a neck portion 40 that upstands from the upper surface 39a. The neck portion 40 passes through an aperture 41 formed in the sheet 31a and stands proud of the outer surface of the sheet 31a. The port coupling 18b is held in place by a heat seal formed between the underside of the sheet 31a and the upper surface 39a of the base portion 39. The outlet 18a comprises a first outlet portion 18a₁, that is relatively narrow and extends from the distal end of the neck portion 40, through the neck portion 40 and partway through the base portion 39, and a second outlet portion 18a₂ that is relatively wide and extends from the first outlet portion 18a₁, to the lower surface 39b of the base portion 39. It will be appreciated that the inwardly facing opening to the outlet 18a has a substantially larger area than that of the aperture 41.

Referring to Figure 5, the second cell-culture-bag 10 further comprises a plurality of filter members 42, 43, 44, each being associated with a respective outlet port 18, 20, 22. The filter members 42, 43, 44 are made from a material that is non-toxic to the MSCs and that does not provide an attractive site for attachment of the MSCs. The filter material should be far less attractive for attachment than the microcarriers. In this embodiment, SEFAR MEDIFAB ® fabrics consisting of monofilament yarns, typically polyester (PET) and polyamide (PA) are suitable. The most preferred is filter 03-15/10 "SEFAR MEDIFAB ® Polyamide" which together with the sheet 31a made from EVA may be fused together during heat sealing to create a high quality seal 45. The first and second filter members 42, 43 have a relatively fine mesh size of 15µm that is intended to allow the passage of red blood cells but block passage of MSCs. The third filter member 44 has a relatively coarse mesh of 60µm that is intended to block the passage of microcarriers. Each filter is heat sealed to the underside of the sheet 31a so as to sealingly enclose the associated port and isolate it from the rest of the chamber 14. This is achieved in the same manner for each port and again, for the purpose of explanation, reference is made to Figure 6 which shows the seeding port 18. The filter member 42 has a surface area which is substantially greater than both the aperture 41 and the inwardly facing opening to the outlet 18a. The filter member 42 is heat sealed to the underside of the sheet 31a such that the outlet port 18 is located centrally with respect to the filter member 42. The seal 45 runs around the perimeter of the filter member 42 and includes localised raised (in a downward direction) portions or protuberances 45a in the underside of the sheet 31a formed during the formation of the seal 45 during heat sealing. Since the filter member 42 is welded to the protuberances 45a, there is created a small clearance space between the filter member 42 and the lower surface 39b of the base portion 39.

The second cell-culture-bag 10 also include some guide holes 46 punched in the skirt 37 which are intended to cooperate with guide rods (not shown) in the rocker device 81 and prevent the cell-culture-bag 10 from being loaded into the rocker device 81 in the incorrect orientation. The second cell-culture-bag 10 further comprises guide pipes 47 that are located within the loop of the skirt 37. The pipes 47 are intended to cooperate with support rods (not shown) in the rocker device 81 which retain the bag in place when it is being rocked. The support rods are able to pass through the pipes 47 via slits (not shown) cut into the sheet 31a. It will have been noticed that the pipes 47 have been arranged to be clear of the clamping region 23.

In use, the second cell-culture-bag 10 performs essentially the same function as the first cell-culture-bag 10 within the system 5. The second cell-culture-bag 10 is loaded into the rocker device 81 and the ports 16, 18, 20, 22 connected up to the perfusion apparatus 50 as shown in Figure 1. One experimental session using the second cell-culture-bag 10 having, as stated above, first and second filter members 42,43 with a mesh size of 15 µm is now described. After pre-filtration to remove aggregates bigger than 100 µm, the crude biopsy aspirate was transferred to the second cell-culture-bag 10. By means of perfusion through the cell-culture-bag, red blood cells and other cell and cell fragments (< 15 µm) are pressed through the 15 µm filter while viable nucleated cells (> 15 µm) are maintained in the cell-culture-bag. The process settings used for the filtration are represented in table 1.

**Table 1: Settings used to filter out red blood cells**

| Settings used to filter out red blood cells | | |
|---|---|---|
| Parameter | Value | Unit |
| Perfusion pump | 7 | ml/min |
| Volume medium bottle | 900 | ml |
| Perfusion time | 129 | min |
| Rocking angle | 165 (+82.5 and-82.5) | deg. |
| Rocking rate | 9 | 1°/sec |
| Acceleration | 34 | 1/s2 |
| Decceleration | 34 | 1/s2 |

The second cell-culture-bag 10 is fed with standard medium from the medium bottle. The standard medium is saturated with O₂ and CO₂ through the oxygenator. The filtrate exiting the cell-culture-bag 10 contains RBCs which are collected in the waste-bottle.

During perfusion the amount of RBCs in the second cell-culture-bag 10 decreased which can be seen by the decrease of the red colour. After two hours filtration, the filtrate (waste bottle) and the residue (cell-culture-bag) were visually inspected using the invert microscope. The filtrate contained a high fraction of RBCs. The amount of RBCs in the residue decreased significantly while the nucleated cells are attached to the microcarriers.

In the initial set-up mode, the perfusion apparatus 50 activates the bag clamp 83 which pinches the sheets 31a, 31b together in the clamping region 23, thereby partitioning the chamber 14 into the two sub-chambers 14a, 14b. The perfusion apparatus 50 is further operable to pump microcarriers from the microcarrier suspension vessel 59 into the sub-chamber 14a. During the set-up mode, either before or after the introduction of microcarriers into the sub-chamber 14a, the user injects by syringe crude bone marrow aspirate into the sub-chamber 14a via the side conduit 71a. The coarse mesh filter 71b serves to block the passage of large blood clots and other large tissue particulates such as bone chips and the like.

Next, the perfusion apparatus 50 is switched into a seeding mode in which the first inlet 16 and the seeding outlet port 18 are in operative connection with a seeding circuit 51 of the perfusion apparatus 50. In this mode, medium is pumped through the sub-chamber 14a and the rocker device 81 subjects the cell-culture-bag 10 to a gentle rocking motion to keep the microcarriers in suspension. In this mode, the effective working volume of the cell-culture-bag 10 is limited to only the sub-chamber 14a, which enables the effective concentration of microcarriers in the sub-chamber 14a to exceed a threshold at which the MSCs of the bone marrow aspirate readily adhere to the microcarriers. Referring to Figure 6, the first filter member 42 having a mesh size of 15µm, allows the red blood cells and other small cells to pass out of the seeding port 18, but blocks the passage of MSCs that have adhered to the microcarriers. Referring to Figure 6, the clearance space 48 between the first filter member 42 and the seeding port coupling 16b ensures that during the seeding operation substantially the whole surface area of the filter member 42 is active in filtering. Both the maintenance of a large active surface area during filtering, and the selection of the filter material which is less attractive for MSC attachment than the microcarriers, serve to militate against clogging at the filter member.

Next, the perfusion apparatus 50 is switched into the expansion mode in which the bag clamp 83 is released, thereby restoring the chamber 14 and the full working volume of the bag, and the expansion circuit 52 is established between the first inlet port 16 and the expansion port 20. In this mode, a more vigorous rocking motion (than in the seeding mode) is required to prevent formation of microcarrier/cell aggregates. Under these conditions, the fresh nutrient being pumped from the expansion medium vessel 57 enables cell expansion to take place. The adherent MSCs are confined to the bag by the second filter member 43. Both the maintenance of a large active surface area during filtering, and the selection of the filter material which is less attractive for MSC attachment than the microcarriers serve to militate against clogging at the filter member.

Next, the perfusion apparatus 50 enters into a harvesting mode in which the first inlet port 16 is in operative connection with a harvesting section 53 of the perfusion apparatus 50. In this mode, the cell disassociating solution is pumped into the chamber 14 and the rocker device 81 subjects the cell-culture-bag 10 to a relatively vigorous rocking motion in order to facilitate detachment of the MSCs from the microcarriers. Under these conditions, the MSCs become detached from the microcarriers. Next, the perfusion apparatus 50 then enters into a collection mode in which the collection port 22 is switched into operative connection with the collection section 54 of the perfusion apparatus 50. In this mode, the detached MSCs exit from the collection port 22 through the third filter member 44 and enter the bio-separator device 75. The 60µm mesh of the third filter member 44 confines the microcarriers, including most of those that have fragmented, to the chamber 14. In the bio-separator device 75 the MSC suspension is concentrated and then pumped into the sterile clinical device 79 (see Figure 1) for subsequent treatment and use.

Thus, the cell-culture-bag 10 is able to internally host each of the seeding, harvesting, and collection operations performed in the expansion of MSCs from crude biopsy.

A third cell-culture-bag 10 (not shown), which does not form part of the present invention, is identical in construction, possible variations in construction, and use to the second cell-culture-bag 10 except that instead of the second filter member 43 having a relatively fine mesh size of 15µm, it has a relatively coarse mesh size of 60µm that is intended to block the passage of microcarriers, but allows the passage of MSCs and red blood cells.

Having such a relatively coarse meshed filter member guarding the expansion port 20 may be sufficient where the operation of the system 5 ensures that during the expansion operation the quantity of MSCs in the chamber 14 that are un-adhered is relatively low, i.e. less than 25% and preferably less than 10%.

The material used for the sheets 31a, 31b, the material used for the filter members 42, 43, 44 and their mesh sizes, and the structure of the ports 16, 18, 20, 22 and the variations thereon disclosed in relation to the second and third cell-culture-bag apply mutatis mutandis to the first cell-culture-bag.

As described above, the system 5 performs the seeding operation in the sub-chamber 14a and then in one discrete step increases the effective working volume of that of the whole chamber 14. In other embodiments, during the seeding operation, the effective working volume of the chamber/the volume of the sub-chamber 14a can be increased in more than one discrete step. The latter can be achieved by means of a bag clamp device having more than one pair of clamping jaws.

A fourth cell-culture-bag 10 intended for use in the Figure 1 system as a further alternative cell-culture-bag is shown in Figure 7. The fourth cell-culture-bag 10 is identical in construction, possible variation in construction, and use to the second cell-culture-bag 10 except in the respects explicitly mentioned below.

Referring to Figure 7, the fourth cell-culture-bag 10 comprises, instead of 3 outlet ports, only two outlet ports, namely a first port serving as a combined seeding/expansion port 19 and a second/collection outlet port 22. A filter member 42 is associated with the seeding/expansion port 19 and has a relatively fine mesh size of 15µm that is intended to allow the passage of red blood cells but block passage of MSCs. A filter member 44 is associated with the collection port 22 and has a relatively course size of 60µm that is intended to block the passage of microcarriers. The filter members 42, 44 are attached around the ports 19, 22 respectively as shown in Figure 6. It will be noted that, in this embodiment, the seeding/expansion port 19 and the collection port 22 are located in close proximity to the first inlet port 16.

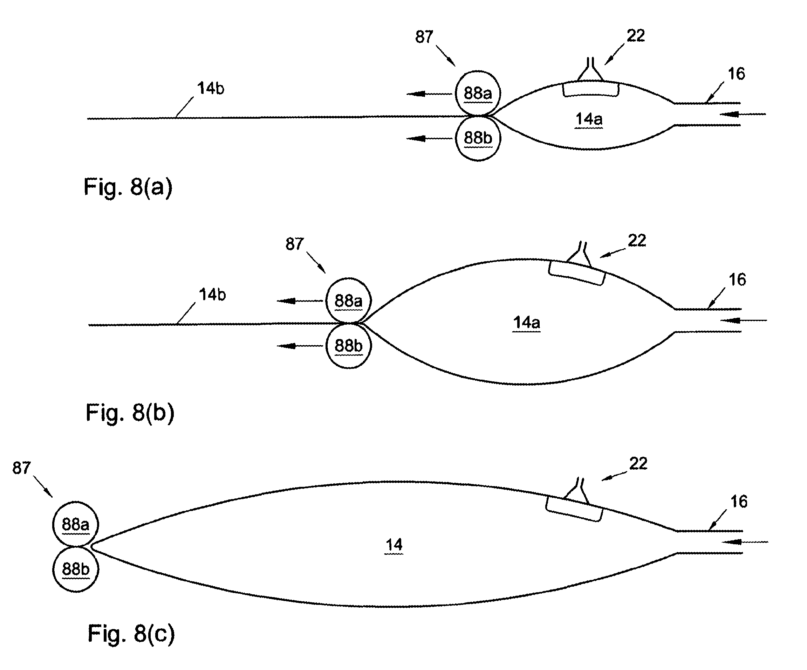

In use, the fourth cell-culture-bag 10 performs essentially the same function as the second cell-culture-bag 10 within the system 5. The fourth cell-culture-bag 10 is loaded into the rocker device 81 (omitted in Figures 7 and 8(a-c)) and the ports 16, 18, 22 connected up to the perfusion apparatus 50. For use with this fourth cell-culture-bag 10 the perfusion apparatus 50 is modified such that the bag clamp device 83 is replaced with a soft roller system 87 as shown in Figures 8(a-c).

Referring to Figure 8(a), in the initial set-up mode, the perfusion apparatus 50 activates the soft roller system 87 which pinches the sheets 31a, 31b between its rollers 88a, 88b, thereby partitioning the chamber 14 into a first, working sub-chambers 14a, and a second sub-chamber 14b. The perfusion apparatus 50 is further operable to pump microcarriers from the microcarrier suspension vessel 59 into the sub-chamber 14a. During the set-up mode, either before or after the introduction of microcarriers into the sub-chamber 14a, the user injects by syringe crude bone marrow aspirate into the sub-chamber 14a via the side conduit 71a (omitted in Figures 7 and 8(a-c)). The coarse mesh filter 71b serves to block the passage of large blood clots and other large tissue particulates such as bone chips and the like.

Next, the perfusion apparatus 50 is switched into a seeding mode in which the first inlet 16 and the seeding/expansion outlet port 19 are in operative connection with a seeding circuit 51 of the perfusion apparatus 50. In this mode, medium is pumped through the sub-chamber 14a and the rocker device 81 subjects the fourth cell-culture-bag 10 to a gentle rocking motion to keep the microcarriers in suspension. In this mode, the effective working volume of the cell-culture-bag 10 is limited to only the sub-chamber 14a, which enables the effective concentration of microcarriers in the sub-chamber 14a to exceed a threshold at which the MSCs of the bone marrow aspirate readily adhere to the microcarriers. The filter member 42 having a mesh size of 15µm, allows the red blood cells and other small cells to pass out of the seeding/expansion port 19, but blocks the passage of MSCs that have adhered to the microcarriers.

Next, referring to Figure 8(b), the perfusion apparatus 50 is switched into the expansion mode in which the expansion circuit is connected across the first inlet port 16 and the seeding/expansion port 19. During the expansion mode, the rollers 88a, 88b are slowly moved, either continuously or in small increments, leftwards as illustrated by the arrows i in Figure 7, so as to gradually increase the volume of sub-chamber 14a/the working volume of the bag. In this mode, a more vigorous rocking motion (than in the seeding mode) is required to prevent formation of microcarrier/cell aggregates. Under these conditions, the fresh nutrient being pumped from the expansion medium vessel 57 enables cell expansion to take place. The gradual increase in the volume of the sub-chamber 14a enables the concentration of cells in the expansion medium to be kept within a favourable range for cell expansion. Eventually, the position in Figure 8(c) is reached.

Next, the perfusion apparatus 50 enters into a harvesting mode in which the first inlet port 16 is in operative connection with a harvesting section 53 of the perfusion apparatus 50. In this mode, the cell disassociating solution is pumped into the chamber 14 and the rocker device 81 subjects the cell-culture-bag 10 to a relatively vigorous rocking motion in order to facilitate detachment of the MSCs from the microcarriers. Under these conditions, the MSCs become detached from the microcarriers. Next, the perfusion apparatus 50 then enters into a collection mode in which the collection port 22 is switched into operative connection with the collection section 54 of the perfusion apparatus 50. In this mode, the detached MSCs exit from the collection port 22 through the filter member 44 and enter the bio-separator device 75. The 60µm mesh of the filter member 44 confines the microcarriers, including most of those that have fragmented, to the chamber 14. In the bio-separator device 75 the MSC suspension is concentrated and then pumped into the sterile clinical device 79 (see Figure 1) for subsequent treatment and use.

Thus, the cell-culture-bag 10 is able to internally host each of the seeding, harvesting, and collection operations performed in the expansion of MSCs from crude biopsy.

In other embodiments, instead of rocker device, a rotation device is used to facilitate the detachment of the MSCs from the microcarriers.

### List of Parts

| | |
|---|---|
| System | 5 |
| Cell-culture-bag | 10 |
| Upper wall portion | 12a |
| Lower wall portion | 12b |
| Chamber | 14 |
| Smaller sub-chamber | 14a |
| Larger sub-chamber | 14b |
| First inlet port | 16 |
| First inlet | 16a |
| First inlet coupling | 16b |
| First/seeding outlet port | 18 |
| First/seeding outlet | 18a |
| First outlet portion | 18a₁ |
| Second outlet portion | 18a₂ |
| First/seeding outlet coupling | 18b |
| First/seeding/expansion outlet port | 19 |
| First/seeding/expansion outlet | 19a |
| First/seeding/expansion outlet coupling | 19b |
| Third/expansion outlet port | 20 |
| Third/expansion outlet | 20a |
| Third/expansion outlet coupling | 20b |
| Second/collection outlet port | 22 |
| Second/collection outlet | 22a |
| Second/collection outlet coupling | 22b |
| Clamping region | 23 |
| Inner filter bag | 25 |
| Outer filter bag | 27 |
| Sheets | 31a, 31b |
| First contour weld | 33 |
| Second contour weld | 35 |
| Looped skirt | 37 |
| Base portion | 39 |
| Upper surface | 39a |
| Lower surface | 39b |
| Neck portion | 40 |
| Aperture | 41 |
| First, second, third filter members | 42, 43, 44 |
| Seal | 45 |
| Guide holes | 46 |
| Guide pipes | 47 |
| Clearance space | 48 |
| Perfusion apparatus | 50 |
| Seeding circuit | 51 |
| Expansion circuit | 52 |
| Harvesting section | 53 |
| Collection section | 54 |
| Controller | 55 |
| Expansion medium vessel | 57 |
| Microcarrier suspension vessel | 59 |
| Cell dissociating solution vessel | 61 |
| Pump | 63 |
| Control device | 65 |
| Oxygen control flow cell | 67a, 67b |
| Ph control flow cell | 69a, 69b |
| Inlet conduit | 71 |
| Side conduit | 71a |
| Filter | 71b |
| Waste medium vessel | 73 |
| Bio-separation device | 75 |
| Pump | 77 |
| Sterile clinical device | 79 |
| Rocker device | 81 |
| Bag clamp device | 83 |
| Circuit clamps | 85 |
| Soft roller system | 87 |
| Rollers | 88a, 88b |

### Experimental Data

To demonstrate the technical significance and non-arbitrary nature of the preferred 8-20µm mesh size of the filter arrangement at the first outlet, the following experimental data is included.

### Filtering of crude biopsy through a 5 µm filter and a 15 µm filter.

Crude bone marrow biopsy from which large particles such as bone chips and fat globules are removed is diluted in 2D human mesenchymal stem cells expansion medium comparatively to the dilution which was obtained during the filtration in a cell-culture-bag 10.

| **Analysis of the crude human bone-marrow biopsy used for filtration through 5 micron vs. 15 micron filter** | | |
|---|---|---|
| **Parameter** | **Value** | **Unit** |
| Volume | 4.2 | ml |
| Nucleated cell concentration | 5,63E+06 | cells/ml |
| Nucleated cells total | 2,37E+07 | cells total |
| Volume cell suspension to be filtrated | 50 | ml |
| X nucleated cells for filtration | 4,66E+06 | cells |
| Volume crude human bone-marrow biopsy needed for filtration | 828 | µl |

The diluted biopsy was divided over two 50 ml syringes which were applied as funnels and were connected to the 5 and the 15 micron filter houses. The cell suspension was filtrated by gravity and the filtration period was monitored. In another experiment the diluted crude-human-bone-marrow biopsy was divided over two 50 ml syringes connected to the 5 and the 15 micron filter houses. By adding manual pressure, the entire cell-suspensions were filtrated. The amount of red blood cells in the filtrate was determined by cell counting using the Burker-Türk hemocytometer. After filtration, the residue was obtained by means of back-flushing the filters with 50 ml 2D human mesenchymal stem cells expansion medium. The filtrate and the residue from both filters were cultured in 2D culture flasks. After 15 days culture the 2D culture flasks were harvested for cell counting to determine human mesenchymal stem cells loss due to filtration. As control, 828 µl of non-filtrated biopsy was re-suspended in 50 ml 2D Human mesenchymal stem cells expansion medium and cultured simultaneously.

### Results and discussion

### Filtration by gravity

The filtration period was monitored and is represented in attachment 1 and table 2:

**Table 2: Filtration by gravity**

| **Filtration by gravity** | | | |
|---|---|---|---|
| **5 micron filter** | | **15 micron filter** | |
| Time (h:m:s) | Volume filtrated (ml) | Time (h:m:s) | Volume filtrated (ml) |
| 00:13:29 | 11 | 00:10:51 | 42 |
| 00:22:07 | 12 | 00:19:28 | 45 |
| 00:33:48 | 14 | 00:31:09 | 48 |
| 00:42:37 | 15 | 00:39:59 | >49 |
| 00:52:41 | 16 | 00:50:02 | >49 |
| 01:30:25 | 18 | 01:27:47 | 50 |
| 02:17:29 | 19 | 02:14:50 | 50 |
| 17:22:44 | 31 | 17:20:05 | 50 |
| 19:52:27 | 35 | 19:49:49 | 50 |

Based on the results represented in table 2 it may be concluded that the filtration period needed to filter a crude-bone-marrow biopsy by gravity using a 15 micron filter is significantly shorter than the filtration period needed using a 5 micron filter.

After a filtration period of 00:10:51, 42 ml of the cell suspension was filtrated through the 15 micron filter. After a filtration period of 19:52:27, 35 ml of the cell suspension was filtrated through the 5 micron filter. This indicates that the filtration period using the 15 micron filter is at least 110 times faster compared to the 5 micron filter. Thus after 1 hour all of the cell suspension was filtered with the 15 micron filter while only 16 ml, which is less than one third of the total volume was filtered with the 5 micron filter. Even after 20 hours still not all of the cell suspension had passed the filter.

When using a volume of about 500 ml, which is the suitable volume to expand human mesenchymal stem cells, e.g. with the cell culture bag 10, using a 15 micron filter, the filtration period is about 2 hours to filter out approximately 90% of the red blood cells. When using a 5 micron filter, the filtration period would at least be increased to 219 hours (= at least 9 days). The relatively long filtration period would increase the process time to obtain expanded human mesenchymal stem cells for clinical applications. In addition, cell viability will most likely be negatively influenced by the relatively long filtration period. This result indicates that the 5 micron filter is unsuitable to filter a crude-human-bone-marrow biopsy.

### Filtration by adding manual pressure

During filtration, a relatively high pressure was needed to filter the crude human bone-marrow biopsy through the 5 micron filter. After filtration the amount of red blood cells in the filtrate was determined by diluting the filtrate 10 times with PBS. The yield of Red blood cells obtained in the filtrate was comparable for both filter, see table 3

**Table 3: Yield of Red blood cells obtained in the filtrate obtained by filtration by adding manual pressure**

| **Yield of Red blood cells obtained in the filtrate obtained by filtration by adding manual pressure** | | |
|---|---|---|
| **Amount of Red blood cells in filtrate 5 micron filter** | | |
| **Parameter** | **Value** | **Unit** |
| Sample 1 | 7,20E+07 | Red blood cells |
| Sample 2 | 6,25E+07 | Red blood cells |
| **Avarage** | **6,73E+07** | **Red** |
| | | **blood cells** |

| **Amount of Red blood cells in filtrate 15 micron filter** | | |
|---|---|---|
| **Parameter** | **Value** | **Unit** |
| Sample 1 | 6,68E+07 | Red blood cells |
| Sample 2 | 6,98E+07 | Red blood cells |
| **Avarage** | **6,83E+07** | **Red blood cells** |

After filtration the following 2D cultures were cultivated:

**Table 4: 2D cultures after filtration**

| **2D cultures to determine human mesenchymal stem cells loss due to filtration** | | |
|---|---|---|
| **5 micron filter** | | |
| **T-flask** | **Cell-suspension** | **Volume (ml)** |
| T-175 | Filtrate | 50 |
| T-175 | Residue | 50 |

| 15 micron filter | | |
|---|---|---|
| **T-flask** | **Cell-suspension** | **Volume (ml)** |
| T-175 | Filtrate | 50 |
| T-175 | Residue | 50 |
| **Control** | | |

| **T-flask** | **Cell-suspension** | **Volume (ml)** |
|---|---|---|
| T-175 | 828 µl Unfiltrated biopsy | 50 |

After 15 days culture the 2D culture flask were harvested for cell counting to determine human mesenchymal stem cells loss due to filtration. The results are represented in table 5.

**Table 5: percentage human mesenchymal stem cells loss due to filtration based on 2D cultures described in table 4**

| **human mesenchymal stem cells loss due to filtration based on 2D cultures** | | |
|---|---|---|
| **Control** | | |
| x cells/ml counted | average | x cells total |
| 1,47E+05 | 1,50E+05 | 1,35E+06 |
| 1,53E+05 | | |
| 1,49E+05 | | |
| **5 micron filter** | | |
| **Residue** | | |

| x cells/ml counted | average | cells total |
|---|---|---|
| 5,60E+03 | 5,20E+03 | 4,68E+04 |
| 5,52E+03 | | |
| 4,48E+03 | | |
| **Filtrate** | | |

| x cells/ml counted | average | cells total |
|---|---|---|
| 7,60E+02 | 8,53E+02 | 7,68E+03 |
| 1,24E+03 | | |
| 5,60E+02 | | |
| **Percentage filtrate** | **14,1** | % |
| **Percentage residue compared to control** | **3,5** | **%** |
| **human mesenchymal stem cells loss** | **96.5** | **%** |
| **15 micron filter** | | |
| **Residue** | | |

| x cells/ml counted | average | cells total |
|---|---|---|
| 7,75E+04 | 7,90E+04 | 7,11E+05 |
| 7,82E+04 | | |
| 8,13E+04 | | |
| **Filtrate** | | |

| x cells/ml counted | average | cells total |
|---|---|---|
| 1,38E+04 | 1,44E+04 | 1,30E+05 |
| 1,40E+04 | | |
| 1,54E+04 | | |
| **Percentage filtrate** | **15,5** | **%** |
| **Percentage residue compared to control** | **52,8** | **%** |
| **human mesenchymal stem cells loss** | **47,2** | % |

The results represented in table 5 indicate that:
- A comparable percentage of human mesenchymal stem cells were obtained in the filtrate (14.1% for the 5 micron filter and 15.5% for the 15 micron filter)
- A higher cell yield was obtained in the residue of the 15 micron filter compared to the residue of the 5 micron filter (52.8% for the 15 micron filter compared to the control culture, 3.5% for the 5 micron filter compared to the control culture)

More than 50% compared to the control cells were obtained with the 15 micron filter while only 3.5 % compared to control with the 5 micron filter. This result was confirmed by visual inspection. It should be noted that with a 15 micron filter integrated into the cell-culture-bag 10, the back-flush procedure is not necessary, due to the design of the cell-culture-bag. Therefore, higher human mesenchymal stem cells yield are expected during filtration in the cell-culture-bag 10.

Overall, it may be concluded that the 15 micron filter is suitable for the filtration of a crude-human-bone-marrow biopsy. The 5 micron filter is not suitable for the filtration of a crude-human-bone-marrow biopsy as it does not result in acceptable yield of viably human mesenchymal stem cells within an acceptable process time.

### Filtration with an 8 micron filter

For the filtration of a cellular-crude-human-bone-marrow-biopsy, BD Falcon cell culture Inserts with an integrated 8 um filter were used. The Physical specifications BD Falcon cell culture Inserts are represented in table 1:

**Table 2.1.1: Physical specifications BD Falcon cell culture Inserts**

| **Physical specifications BD Falcon cell culture Inserts** | |
|---|---|
| **Description** | **Specification for 6-well plate insert** |
| Effective diameter of membrane | 23.1 mm |
| Effective growth area of membrane | 4.2 cm² |
| Insert height | 17.2 mm |
| Distance from membrane to bottom of the well | 0.9 mm |
| Suggested media in insert | 1.5-2.5 ml |
| Suggested media in well | 2.7-3.2 ml |
| Growth area in plate well | 9.6 cm² |
| Material | Track-etched polyethylene terephthalate (PET) |

### Analyses and pre-treatment of the cellular-crude-human-bone-marrow-biopsy

Due to donor variations, the cellular-crude-human-bone-marrow-biopsy was analysed to determine the starting point regarding the total number of nucleated cells. The cellular-crude-human-bone-marrow-biopsy was pre-filtrated though a 100 µm cell-strainer to remove aggregates bigger than 100 µm (eg. fat, tissue and coagulated red blood cells). The filtrate (e.g. non coagulated nucleated cells and red blood cells) was diluted in human mesenchymal stem cells 2D expansion medium at a concentration of 2.50E06 cells/ml.

**Table 2.1.2: Analysis of the cellular-crude-human-bone-marrow-biopsy**

| **Analysis of the cellular-crude-human-bone-marrow-biopsy, experiment 1** | | |
|---|---|---|
| **Parameter** | **Value** | **Unit** |
| Volume | 19 | ml |
| Nucleated cell concentration | 1,49E+07 | cells/ml |
| Nucleated cells total | 2,83E+08 | cells total |
| Dilution @ 2.500E6 cells/ml human mesenchymal stem cells 2D expansion medium | 113 | ml |

### Filtration of the cellular-crude-human-bone-marrow-biopsy

As control to the experimental conditions, the cellular-crude-human-bone-marrow-biopsy, which was only pre-filtrated though a 100 µm cell-strainer to remove aggregates bigger than 100 µm, was cultured along with the experimental was cultured along with the experimental cultures. A fraction of the diluted cellular-crude-human-bone-marrow-biopsy was seeded directly in a 6-well-cultureplate, without filtration through an 8 µm filter. The control culture was not filtrated through an 8 µm filter thus a co-culture of nucleated cells and red blood cells was obtained. After 6 days culture, human mesenchymal stem cells attached to the culture surface of the 6-well-culture-plate, while red blood cells were maintained in suspension. By withdrawing the medium from the 6-well-culture-plate, the human mesenchymal stem cells were physically separated from the red blood cells.

During this experiment, a fraction of the diluted cellular-crude-human-bone-marrow-biopsy was filtrated through an 8 µm filter to physically separate the red blood cells from the human mesenchymal stem cells. The fraction of the diluted cellular-crude-human-bone-marrow-biopsy used for the experimental culture was equal to the fraction used for the control culture.
The materials used for the filtration were 6-well-plate-inserts with integrated 8 µm filter from BD Falcon in combination with 6-well-culture-plates. Blockage of the filter was prevented by filtering dynamically by means of a rocking plateau. The filters containing the diluted cellular-crude-human-bone-marrow-biopsy were placed in the CO2-incubator on a rocking plateau rocking at 5 rpm with an angel of 15 degree.

After filtration, the red blood cells smaller than 8 µm were obtained in the filtrate and the human mesenchymal stem cells bigger than 8 µm were retained by the filter. The residue, containing the human mesenchymal stem cells, was obtained by flushing the filter, 2, 3 and 4 times. The residue was cultured to determine the human mesenchymal stem cells yield. To determine the human mesenchymal stem cells loss in the filtrate, the filtrate was cultured simultaneously. All culture procedures and cell-culture equipment were equal for the control cultures and the experimental cultures.

After 1 hour filtration, a 6-well-culture-plate with the following set-up was incubated:

**Table 2.1.3: arrangement 6-well plates, after 1hour filtration**

| **Arrangement 6-well- culture- plates, after 1hour filtration** | | |
|---|---|---|
| **Control :** unfiltered cell suspension | **filtrate :** red blood cells cell < 8 µm | **Filter:** Remaining human mesenchymal stem cells |
| **Residue after flushing the filter two times:** human mesenchymal stem cells > 8 µm | **Residue after flushing the filter for the third time:** human mesenchymal stem cells > 8 µm | **Residue after flushing the filter for the fourth time:** human mesenchymal stem cells > 8 µm |

All the cultures were refreshed after 6 days culture. By refreshing the medium, the human mesenchymal stem cells from the control culture were physically separated from the red blood cells in suspension.

The 6-well-culture-plates were cultured for 11 days after which the human mesenchymal stem cells yields were determined by harvesting and counting the human mesenchymal stem cells.

### Results and discussion

After 6 days culture, all the cultures were refreshed and visual inspection was performed.

Some colonies and stretched cells were observed in the control culture although the amount was visibly lower than the human mesenchymal stem cells yield obtained from the residue of the filter, after flushing twice. Furthermore, it could be observed that a lot of red blood cells were attached to the colonies of the control culture, resulting in a relatively un-healthy cell-morphology

After 11 days culture the human mesenchymal stem cells yield was determined, see table 2.1.4:

**Table 2.1.4: human mesenchymal stem cells yield control culture and experimental cultures**

| **human mesenchymal stem cells yield** | |
|---|---|
| **Control** | **cells per well** |
| | **5,33E+03** |
| **Experimental cultures :** | **cells per well** |
| Filtrate | 3,30E+02 |
| Residue, after flushing the filter two times | 1,51E+05 |
| Residue, after flushing the filter for the third time | 6,86E+03 |
| Residue, after flushing the filter for the fourth time | 7,36E+03 |
| Well containing filter | 4,63E+03 |
| Filter, after flushing | 7,40E+04 |
| **Total human mesenchymal stem cells yield from filtrated biopsy** | **2,44E+05** |
| **Percentage experimental cultures vs. Total human mesenchymal stem cells yield from filtrated biopsy** | **NA** |
| % Filtrate | 0,1 |
| **% Residue flush 2 times** | **61,9** |
| % Residue flushed 3th time | 2,8 |
| % Residue flushed 4th time | 3,0 |
| % well containing filter | 1,9 |
| **% Filter vs. Total from filter** | **30,3** |
| **% Control vs. Residue, after flushing the filter 2 times** | **3,5** |
| **% Control vs. Total human mesenchymal stem cells yield from filtrated biopsy** | **2,2** |

These results indicate that:
- A negligible fraction of human mesenchymal stem cells were lost in the filtrate (0.1%)
- A high fraction of human mesenchymal stem cells were obtained after flushing the filter twice (61.9 %)
- Negligible fractions of human mesenchymal stem cells were obtained after flushing a third and a fourth time (total 5.8 %)
- However, a high fraction of human mesenchymal stem cells were obtained from the filter (30.3 %), indicating a higher human mesenchymal stem cells yield can be obtained by means of a system in which it is not necessary to flush the filter, like the cell-culture-bag 10.
- The human mesenchymal stem cells yield obtained from the control culture was negligible compared to the amount of human mesenchymal stem cells obtained from the Residue, after flushing the filter 2 times (3.5%)

These observations indicate that it is feasible to physically separate human mesenchymal stem cells (hMSCs) from red blood cells by means of filtration using an 8 µm, without losing hMSCs in the process. The hMSCs yield is increased significantly by isolating hMSCs from a cellular-crude-human-bone-marrow-biopsy prior to culture. The hMSCs yield obtained after flushing the filter two times was 28 fold higher compared to the control culture. In addition, a healthier cell-morphology is obtained.
The hMSCs yield can be further increased by means of a system in which it is not necessary to flush the filter, like the cell-culture-bag 10.

### Expansion in culture bags

In this experiment expansion of cells in 2D culture flask were compared to 3D culture bags. Gas-permeable-cell-culture-bags were used for the 3D cultures on Cytodex 1 microcarriers. The gas-permeable-cell-culture-bags were placed on a rotating platform (which is comparable to a 180 ° rocking angle). The gas-permeable-cell-culture-bag was incubated in a CO2-incubator at 37 °C and 5% CO₂.

### Analyses of the cellular-crude-human-bone-marrow-biopsy

Due to donor variations, the cellular-crude-human-bone-marrow-biopsy was analysed to determine the starting point regarding the total number of nucleated cells. The cellular-crude-human-bone-marrow-biopsy was pre-) filtrated though a 100 µm cell-strainer to remove aggregates bigger than 100 µm (e.g. fat, tissue and coagulated red blood cells). The filtrate (e.g. non coagulated nucleated cells and red blood cells) was diluted in human mesenchymal stem cells 2D expansion medium at a concentration of 2.50E06 cells/ml.

**Table 2.2.1: Analysis of the cellular-crude-human-bone-marrow-biopsy**

| **Analysis of the cellular-crude-human-bone-marrow-biopsy** | | |
|---|---|---|
| **Parameter** | **Value** | **Unit** |
| Volume | 14,35 | ml |
| Nucleated cell concentration | 1,14E+07 | cells/ml |
| Nucleated cells total | 1,92E+08 | cells total |
| Dilution @ 2.500E6 cells/ml human mesenchymal stem cells 2D expansion medium | 76,8 | ml |

### Filtration of the cellular-crude-human-bone-marrow-biopsy

As control to the experimental conditions, the cellular-crude-human-bone-marrow-biopsy, which was pre-filtrated though a 100 µm cell-strainer to ) remove aggregates bigger than 100 µm, was cultured along with the experimental cultures. A fraction of the diluted cellular-crude-human-bone-marrow-biopsy was seeded directly, without filtration through an 8 µm filter, in a T-75 culture flask for the 2D control. For the 3D control culture a fraction of the diluted cellular-crude-human-bone-marrow-biopsy was seeded directly into a gas-permeable-cell-culture-bag containing Cytodex microcarriers, without filtration through an 8 µm filter.
The control cultures were not filtrated through an 8 µm filter thus a co-culture of nucleated cells and red blood cells was obtained. After 6 days culture, human mesenchymal stem cells attached to the culture surface of the T-75 culture flask or Cytodex 1 microcarriers, while red blood cells were maintained in suspension. By withdrawing the medium from the cultures, the human mesenchymal stem cells were physically separated from the red blood cells. The fractions of the diluted cellular-crude-human-bone-marrow-biopsy used for control cultures were equal to the fractions used for the experimental cultures. All culture procedures and cell-culture equipment were equal for the control cultures and the experimental cultures.

During this experiment, a fraction of the diluted cellular-crude-human-bone-marrow-biopsy was filtrated through an 8 µm filter to physically separate the red blood cells from the mesenchymal stem cells. The materials used for the filtration were 6-well-plate-inserts with integrated 8 µm filter from BD Falcon in combination with 6-well-culture-plates. Blockage of the filter was prevented by filtering dynamically by means of a rocking plateau. The filters containing the diluted cellular-crude-human-bone-marrow-biopsy were placed in the CO2-incubator on a rocking plateau rocking at 5 rpm with an angel of 15 degree. After filtration, the red blood cells smaller than 8 µm were obtained in the filtrate and the human mesenchymal stem cells bigger than 8 µm were blocked by the filter. The residue, containing the human mesenchymal stem cells, was obtained by flushing the filter two times.
The residue was cultured in a T-75 culture flask for the 2D control and in a gas-permeable-cell-culture-bag cultured in a CO2-incubator containing Cytodex microcarriers for the 3D control.

In addition, a fraction of the diluted cellular-crude-human-bone-marrow-biopsy was used to isolate the human mesenchymal stem cells by means of centrifugation. The centrifuge works using the sedimentation principle, where the centripetal acceleration causes particles with a relatively high density (e.g. human mesenchymal stem cells) to separate out along the radial direction (the bottom of the centrifuge tube). By the same token, particles with a relatively low density (e.g. red blood cells) will tend to move to the top. By subtracting the top layer, red blood cells and human mesenchymal stem cells were psychically separated.
The remainder suspension from the bottom of the centrifuge tube was re-suspended. The obtained cell-suspension was cultured in T-75 culture flask for the 2D control and in a gas-permeable-cell-culture-bag containing Cytodex microcarriers for the 3D control.

In summary, human mesenchymal stem cells were isolated and cultured accordingly:

**Isolation and culture conditions**

| **Isolation and culture conditions** | | |
|---|---|---|
| **Condition** | **3D (10 ml** gas-permeable-cell-culture-bag) | **2D (15 ml T-75 culture flask)** |
| 1 | Control | Control |
| 2 | Filtrated culture | Filtrated culture |
| 3 | Centrifuged culture | Centrifuged culture |

The following parameters were applied:

| **Parameters applied during** | |
|---|---|
| **2D:** | **Standard conditions** |
| **Parameters:** | **Setting:** |
| Starting volume: | 15 ml |
| Seeding density: | 5.00E05 cells / 0.2 ml |

**Parameters and settings experiment 2**

| | |
|---|---|
| First refreshment | After 6 days |
| Second refreshment | 3-4 days |

| **3D:** | |
|---|---|
| **Parameters:** | **Setting:** |
| Starting volume: | 10 ml |
| Microcarrier density: | 20 cm² microcarrier / ml medium |
| Rocking regime during seeding: | Rotating continuously at minimum rocking rate (+/- 5 rpm) |
| Seeding density: | 5.00E05 cells / 0.2 ml → excluding cell loss due to treatment |
| First refreshment | After 6 days |
| Second refreshment | 3-4 days |

Cell attachment and growth in the cell-culture-bags were monitored by means of visual inspection.

### Results and discussion

After 6 days culture, all the cultures were refreshed and visual inspection was performed.

Visual inspection of the 2D cultures indicated that the highest human mesenchymal stem cells yield and the most viable human mesenchymal stem cells morphology were obtained in the filtrated culture. It was visible that the amount of red blood cells decreased by washing, however, the filtering procedure was clearly more effective.

Visual inspection of the 3D cultures indicated that the highest human mesenchymal stem cells yield and the most viable morphology were obtained in the filtrated culture. Cell-aggregates were formed in the control culture and the centrifuged culture, even though the amount of red blood cells was reduced by centrifugation. These observations indicate that human mesenchymal stem cells need to be isolated completely prior to 3D culture using Cytodex 1 microcarriers.

After 10 days culture, all the cultures were refreshed and visual inspection was performed.

Visual inspection of the 2D cultures indicated human mesenchymal stem cells expansion in all cultures. However, the highest human mesenchymal stem cells yield and the most viable morphology were obtained in the filtrated culture. Confirming the results obtained during experiment 1 described in section 2.1.

Visual inspection of the 3D cultures indicated expansion of cell-aggregates in the control culture and the centrifuged culture. No stretched human mesenchymal stem cells were observed in contrast to the filtrated culture were a lot of healthy stretched human mesenchymal stem cells were observed.

These observations indicate that in prior art procedures, human mesenchymal stem cells need to be isolated completely prior to 3D culture using Cytodex 1 microcarriers. By means of the standard 2D isolation procedure based on adherence this means that a 2D isolation step of 6 days is needed prior to 3D culture on Cytodex microcarriers. The isolation of mesenchymal stem cells can be obviated by using a filter between 8 and 20µm and expansion of crude cellular biopsies is feasible to culture directly on Cytodex 1 microcarriers.

### Filtration of a crude-human-bone-marrow-biopsy through a 40 micron filter.

The results described above indicated that is not feasible to physically separate human mesenchymal stem cells from red blood cells by means of filtration using a 100 µm. In addition it has been demonstrated that the human mesenchymal stem cells yield is increased significantly by isolating the human mesenchymal stem cells from a cellular-crude-human-bone-marrow-biopsy prior to culture.

### Procedure

A crude-human-bone-marrow-biopsy was filtrated through a 40 micron filter. Before and after filtration, visual inspection was performed. In addition, the amount of nucleated cells in the biopsy was counted before and after filtration. This was done to indicate the efficiency of the filtration procedure using a 40 micron filter.

### Results and discussion

Before and after filtration, visual inspection was performed to visualize the cellular mixture of wanted cells (human mesenchymal stem cells) and unwanted cells (e.g. red blood cells).

Visual inspection clearly indicates that is not feasible to physically separate human mesenchymal stem cells from red blood cells by means of filtration using a 40 µm.

These observations were confirmed by cell counting since the amount of nucleated cells before filtration was comparable to the amount of nucleated cells after filtration, see table 2.3.1.

| **Cell counting before and after filtration of a crude-human-bone-marrow-biopsy through a 40 micron filter** | | |
|---|---|---|
| **Parameter** | **Value** | **Unit** |
| Before filtration | 6,98E+07 | cells total |
| After filtration | 6,75E+07 | cells total |
| **Percentage nucleated cells isolated by filtration** | **3,2** | % |

The results obtained by cell counting clearly indicate that a negligible amount of nucleated cells were isolated after filtration through a 40 micron filter. From these results can be concluded that filtration crude-human-bone-marrow-biopsy through a 40 micron filter is not sufficient for the isolation of human mesenchymal stem cells.

### Conclusions

The described results indicate that:
- It is not feasible to physically separate human mesenchymal stem cells from red blood cells by means of filtration using a 40-100 µm filter.
- It is feasible to physically separate human mesenchymal stem cells from red blood cells by means of filtration using an 8 µm, without losing human mesenchymal stem cells in the process.
- The hypothesis that the standard 2D culture procedure may be optimized by separating the human mesenchymal stem cells from the red blood cells prior to culture was confirmed. The human mesenchymal stem cells yield is increased significantly by isolating the human mesenchymal stem cells from a cellular-crude-human-bone-marrow-biopsy prior to culture. The human mesenchymal stem cells yield obtained after flushing the filter two times was 28 fold higher compared to the control culture. In addition, a healthier cell-morphology is obtained.
- Since the filter was not flushed effectively, the human mesenchymal stem cells yield can be further increased by means of a system in which it is not necessary to flush the filter, like the cell-culture-bag 10.
- By means of centrifugation the concentration of red blood cells can be decreased. However, the filtering procedure was clearly more effective.
- Cell-aggregates were formed in the control culture and the centrifuged culture, even though the amount of red blood cells was reduced by centrifugation. No stretched human mesenchymal stem cells were observed in contrast to the filtrated culture were a lot of healthy stretched human mesenchymal stem cells were observed.
- Therefore it can be concluded that red blood cells need to be removed prior to 3D culture using Cytodex 1 microcarriers.
- By means of the standard 2D isolation procedure based on adherence this means that a 2D isolation step of 6 days is needed prior to 3D culture on Cytodex microcarriers. By means of filtration it is feasible to culture directly on Cytodex 1 microcarriers.
- Using the filtering with a poresize of 8-20 µm crude cellular biopsies may be cultured directly in a 3D expansion vessel, such as the cell-culture-bag 10. Filtering removes the need for preculture in the standard 2D procedure which takes about 6 days to remove the red blood cells. With filtering through a poresize of 8-20 µm the process time and process steps are decreased while the human mesenchymal stem cells yield is increased.

## Claims

1. A system for expanding stem cells from a crude biopsy, comprising a cell-culture-bag (10) and a perfusion apparatus (50), wherein
the cell-culture-bag (10), comprises: outer walls; a chamber (14) located within the walls; a first inlet (16) and first and second outlets (19, 22) located at one end of the bag (10); and a filter arrangement (42, 44) integral to the cell-culture-bag and associated with the first and second outlets, wherein the filter arrangement (42, 44) is constructed to allow passage of red blood cells and block passage of stem cells from the first outlet (19), and block passage of microcarriers from the second outlet (22), wherein the filter arrangement (42, 44) filters the fluid path from the chamber to the first outlet (19) with a fine mesh of 8-20µm, wherein the filter arrangement filters (42, 44) the fluid path from the chamber to the second outlet (22) with a coarse mesh of 50-100µm; and
the perfusion apparatus (50) is connected to the first inlet and the first and second outlets for performing seeding, expansion, harvesting and collection operations on stem cells placed within the chamber (14);
wherein the perfusion apparatus (50) comprises a roller system (87) capable of continuously varying the working volume of the chamber (14).

2. A system as in claim 1, wherein the roller system (87) is capable of increasing the working volume of the chamber (14).

3. A system as in claim 2, wherein the roller system (87) comprises first and second rollers (88a, 88b).

4. A system as in any preceding claim, wherein the filter arrangement (42, 44) is made from a material which is less attractive for mesenchymal stromal cell attachment than microcarriers.

5. A system as in any preceding claim, wherein the filter arrangement (42, 44) comprises a filter member (42, 44) that is associated with a said outlet (19, 22) and joins to the portion of the outlet wall surrounding the said outlet so as to sealingly enclose said outlet.

6. A system as in claim 5, wherein the filter member (42, 44) has an area which is substantially greater than that of the associated outlet (19, 22).

7. A system as in claim 5 or 6, wherein the joint between the filter member (42, 44) and the outer wall includes a protuberance that creates a small clearance space between the filter member and the outer wall.

8. A cell-culture-bag (10) for use in the system according to any preceding claim.

## Patentansprüche

1. System zum Vermehren von Stammzellen aus einer groben Biopsie, umfassend einen Zellkulturbeutel (10) und ein Perfusionsgerät (50), wobei
der Zellkulturbeutel (10) umfasst: Außenwände; eine Kammer (14), die innerhalb der Wände angeordnet ist; einen ersten Einlass (16) und einen ersten und einen zweiten Auslass (19, 22), die an einem Ende des Beutels (10) angeordnet sind; und eine Filteranordnung (42, 44), die mit dem Zellkulturbeutel einstückig ist und dem ersten und dem zweiten Auslass zugeordnet ist, wobei die Filteranordnung (42, 44) dazu konstruiert ist, Hindurchlassen von roten Blutkörperchen aus dem ersten Auslass (19) zu erlauben und Hindurchlassen von Stammzellen daraus zu blockieren, und Hindurchlassen von Mikroträgern aus dem zweiten Auslass (22) zu blockieren, wobei die Filteranordnung (42, 44) den Flüssigkeitspfad von der Kammer zum ersten Auslass (19) mit einem feinen Netz von 8 - 20 µm filtert, wobei die Filteranordnung (42, 44) den Flüssigkeitspfad von der Kammer zum zweiten Auslass (22) mit einem groben Netz von 50 - 100 µm filtert; und
das Perfusionsgerät (50) mit dem ersten Einlass und dem ersten und dem zweiten Auslass verbunden ist, um Ansetz-, Vermehrungs-, Ernte- und Sammelvorgänge an in der Kammer (14) angeordneten Stammzellen durchzuführen;
wobei das Perfusionsgerät (50) ein Walzensystem (87) umfasst, das dazu fähig ist, das Arbeitsvolumen der Kammer (14) kontinuierlich zu variieren.

2. System gemäß Anspruch 1, wobei das Walzensystem (87) dazu fähig ist, das Arbeitsvolumen der Kammer (14) zu vergrößern.

3. System gemäß Anspruch 2, wobei das Walzensystem (87) eine erste und eine zweite Walze (88a, 88b) umfasst.

4. System gemäß einem der vorhergehenden Ansprüche, wobei die Filteranordnung (42, 44) aus einem Material hergestellt ist, das für eine Anheftung von mesenchymalen Stammzellen weniger attraktiv ist als von Mikroträgern.

5. System gemäß einem der vorhergehenden Ansprüche, wobei die Filteranordnung (42, 44) ein Filterelement (42, 44) umfasst, das dem Auslass (19, 22) zugeordnet ist und mit dem Teil der Auslasswand verbunden ist, die den Auslass so umgibt, um den Auslass dichtend einzuschließen.

6. System gemäß Anspruch 5, wobei das Filterelement (42, 44) eine Fläche hat, die wesentlich größer als diejenige des zugeordneten Auslasses (19, 22) ist.

7. System gemäß Anspruch 5 oder 6, wobei die Verbindung zwischen dem Filterelement (42, 44) und der Außenwand einen Fortsatz aufweist, der einen kleinen Zwischenraum zwischen dem Filterelement und der Außenwand schafft.

8. Zellkulturbeutel (10) zur Verwendung in dem System gemäß einem der vorhergehenden Ansprüche.

## Revendications

1. Système pour l'expansion des cellules souches à partir d'une biopsie brute, comprenant une poche de culture cellulaire (10) et un appareil de perfusion (50), dans lequel :
la poche de culture cellulaire (10) comprend : des parois externes; une chambre (14) positionnée à l'intérieur des parois ; une première entrée (16) et des première et seconde sorties (19, 22) positionnées au niveau d'une extrémité de la poche (10) ; et un agencement de filtre (42, 44) solidaire de la poche de culture cellulaire et associé avec les première et seconde sorties, dans lequel l'agencement de filtre (42, 44) est construit pour permettre le passage des globules rouges et empêcher le passage des cellules souches par la première sortie (19), et empêcher le passage des micro-supports par la seconde sortie (22), dans lequel l'agencement de filtre (42, 44) filtre la trajectoire de fluide de la chambre à la première sortie (19) avec une maille fine de 8-20 µm, dans lequel l'agencement de filtre (42, 44) filtre la trajectoire de fluide de la chambre à la seconde sortie (22) avec une maille grossière de 50-100 µm ; et
l'appareil de perfusion (50) est raccordé à la première entrée et aux première et seconde sorties pour réaliser les opérations d'ensemencement, d'expansion, de récolte et de collecte sur les cellules souches placées à l'intérieur de la chambre (14) ;
dans lequel l'appareil de perfusion (50) comprend un système de rouleaux (87) pouvant modifier de manière continue le volume de travail de chambre (14).

2. Système selon la revendication 1, dans lequel le système de rouleaux (87) peut augmenter le volume de travail de la chambre (14).

3. Système selon la revendication 2, dans lequel le système de rouleaux (87) comprend des premier et second rouleaux (88a, 88b).

4. Système selon l'une quelconque des revendications précédentes, dans lequel l'agencement de filtre (42, 44) est réalisé à partir d'un matériau qui est moins attractif pour la fixation des cellules mésenchymateuses stromales que les micro-supports.

5. Système selon l'une quelconque des revendications précédentes, dans lequel l'agencement de filtre (42, 44) comprend un élément de filtre (42, 44) qui est associé à ladite sortie (19, 22) et s'assemble à la partie de la paroi de sortie entourant ladite sortie afin d'enfermer de manière étanche ladite sortie.

6. Système selon la revendication 5, dans lequel l'élément de filtre (42, 44) a une surface qui est sensiblement supérieure à celle de la sortie (19, 22) associée.

7. Système selon la revendication 5 ou 6, dans lequel le joint entre l'élément de filtre (42, 44) et la paroi externe comprend une protubérance qui crée un petit espace de jeu entre l'élément de filtre et la paroi externe.

8. Poche de culture cellulaire (10) destinée à être utilisée dans le système selon l'une quelconque des revendications précédentes.
